# EUROPEAN PATENT APPLICATION

(11) **EP 4 696 300 A1**
(43) Date of publication of application: **18.02.2026**
(21) Application number: 24194501.3
(22) Date of filing: 14.08.2024
(51) Int. Cl.: A61K 9/00, A61K 31/164, A61K 31/56, A61K 47/02, A61K 47/10, A61K 47/12, A61K 47/18, A61K 47/26

(54) **AQUEOUS COMPOSITION COMPRISING FLUTICASONE**

(71) Applicant: Marinomed Biotech AG, 2100 Korneuburg (AT)
(72) Inventor: Zemora, Georgeta, 1020 Wien (AT); Winter, Dorian, 1030 Wien (AT); Siegl, Cornelia, 3400 Klosterneuburg (AT); Prieschl-Grassauer, Eva, 1210 Wien (AT)
(74) Representative: Bogensberger, Burkhard

(57) **Abstract**

The invention relates to a buffered aqueous composition comprising fluticasone propionate dissolved therein in a concentration of at least 20 *µ*g/mL, wherein the composition further comprises a saponin component selected from the group consisting of escin, glycyrrhizin, and Quillaja saponaria extract, a buffer adjusted to a pH of from 4 to 8; and the surfactant poloxamer P407 in a concentration of from 2.5 to 15 %w/v.

The invention further relates to the use of the composition in the treatment of inflammatory and/or allergic conditions of the human body.

## Description

### FIELD OF INVENTION

The present invention relates to an aqueous composition comprising fluticasone, for use in the treatment of allergic diseases and inflammatory conditions.

### INTRODUCTION

WO2017009480 discloses a method of substantially increasing the solubility of poorly water-soluble hydrophobic drugs in aqueous solutions. The method comprises dissolving the hydrophobic drug in a suitable organic solvent and mixing the organic solvent with an aqueous buffer solution comprising a saponin component selected from the group of escin, glycyrrhizin and Quillaja saponaria extract, alongwith dexpanthenol and optionally further additives. The solubilization process is based on the formation of saponin micelles in a buffered aqueous environment.

One such poorly water-soluble molecule is fluticasone propionate (FP), a glucocorticoid receptor agonist having antiallergic and antiinflammatory activity, the solubility of which in water being less than 0.2 *µ*g/ml. Attempts to solubilize fluticasone propionate following the method disclosed in WO2017009480, i.e. dissolving FP in a phosphate buffered aqueous solvent comprising escin, glycyrrhizin, polypropylene glycol, and dexpanthenol as essential ingredients, resulted in aqueous preparations comprising at best 15 *µ*g/ml of dissolved FP. The preparations appeared to be stable at room temperature over an extended period of several months.

Yet, the achieved maximum concentration of FP in the aqueous environment was not fully satisfactory. Therefore, further attempts have been made to raise the concentration of dissolved FP drug in an aqueous solvent without impairing the shelf life of the resulting preparation.

### DESCRIPTION OF THE INVENTION

Using saponins as solubilization enhancers for poorly water-soluble drugs in pharmaceutical compositions is just one way of trying to take hydrophic drugs into solution in aqueous solvents. Another approach is to apply surfactants, either as single additives or by way of a mixture of different surfactants.

Given that there existed a need of improving the solubilization system taught in WO2017009480, at least with respect to FP, it was contemplated to try to combine the saponins of WO2017009480 with suitable surfactants and, if necessary or advantageous, also with further ingredients required under the protocol of WO2017009480, in order to enhance the concentration of dissolved drug in a pharmaceutically acceptable aqueous solvent system without impairing shelf live and storage stability. The main incentive for conducting pertinent experimentation was to provide an aqueous pharmaceutical composition comprising FP dissolved therein at a substantially increased concentration, as compared to pure or buffered water as a solvent, in order to allow for administering FP at reduced dosage units and hence reduced toxicity, as compared to commerciallay available FP suspensions, but yet with substantially improved uptake efficiency and tremendously improved physiological efficacy.

The possible fields of application of the envisaged ready-for-use composition comprise its use as an anti-allergic and/or anti-inflammatory remedy, especially as a medicament in the therapeutic treatment of allergic and/or inflammatory conditions of the eye, of the skin, of the respiratory pathways, the lungs, and mucosal tissues of the mouth, the nose and the genital including the anogenital regions. Typically, the present FP preparations may be favorably applied in the treatment of inflammatory diseases such as acute, chronic, or allergic asthma, hayfever, allergic diseases caused by pollen, animal hair or dust particles, conjunctivitis of the eye, but also in the treatment of skin inflammations caused by lichen ruber planus or lichen sclerosus.

One such group of surfactants is the group of poloxamers, which are nonionic poly(ethylene oxide)-poly(propylene oxide) block copolymers (PEO-PPO block copolymers) frequently used in pharmaceutical preparations.They function both as surfactants and drug stabilizing compounds. Poloxamers have also notable physiological properties by themselves and go with only low toxicity, if at all. They are of an amphiphilic nature in aqueous solutions and are able to spontaneously form micelles that can accommodate hydrophobic compounds and thus can serve as solubility enhancers. The amphiphilic character is due to their chemical structure, with the PEO block being mainly hydrophilic and the PPO block being mainly hydrophobic.

According to the FDA guide poloxamers are regarded as inactive ingredients suitable for various types of pharmaceutical compositions including compositions adapted for intravenous, intratympanic, intramuscular, oral, ophthalmic, topical, or periodontal administration. As such, poloxamers can be found in many galenic formulations such as liquids, suspensions, tablets, gels, solutions, creams, capsules, and powders.

Given the hydrophobicity and poor water solubility of fluticasone propionate (FP) commercially available aqueous preparations comprise FP as an aqueous suspension.

Out of a number of different poloxamers obtainable from BASF, Germany, four poloxamers, i.e. poloxamers 124, 188, 338 and 407 (also known under the BASF trade names Kolliphor^{®}, Kollisolv^{®} or Pluronic^{®}), were selected for the purposes of the present invention. Accordingly, where poloxamers are designated K124, K188, K338 or K407 hereinafter these designations are synomymous with the terms P124, P188, P338 or P407.

It turned out that by supplementing the buffered aqueous solvent solution comprising saponins and dexpanthenol disclosed in WO2017009480 with one or more poloxamers, the solubility and stability of FP in the aqueous solvent system was remarkably augmented over the control preprations without poloxamer. Among the poloxamers tested, the poloxamers nos. 338 and 407, each one individually, showed a synergistic effect with the saponin/dexpanthenol solvent system of WO2017009480. The synergistic effect was more pronounced with poloxamer 407 which increased the solubility of fluticasone propionate up to 80 *µ*g/ml and which preparation remained stable for four months at room temperature.

Preparations comprising various concentrations of poloxamer but lacking the saponin and dexpanthenol components did not achieve the highest values of dissolved FP concentrations. It therefore seems that a combination of one or more poloxamer surfactants and one or more saponins, optionally along with dexpanthenol, in an aqueous buffered solution, is required to obtain maximum solubility of FP in an aqueous solvent system. The surprisingly good results achieved with this combination of two different classes of amphiphiles, namely copolymers (e.g. poloxamers) and saponins (e.g. escin, glycyrrhizin, Quillaya saponaria exctract) may be highly promising and may have quite some potential as a carrier system for hydrophobic drugs. Such a combination can be used to form structures with more suitable properties, offering interesting perspectives of application due to increased solubility and stability of hydrophobic drugs in an aqueous formulation, especially but not exclusively applicable to fluticasone propionate FP.

This kind of improved formulation prepared in accordance with the present invention not only allows for enhanced therapeutic efficacy of FP, but also for expanding the options of administration from nasal to buccal, oral, respiratory, ocular and further topical or systemic applications.

In order to further illustrate and facilitate understanding of the present invention the following examples are set forth. They shall not be construed, however, to limit the invention to the specific examples disclosed. Variations of the invention will be apparent to those of skill in the art without deviating from the spirit of the invention.

### BRIEF DESCRIPTION OF THE FIGURES

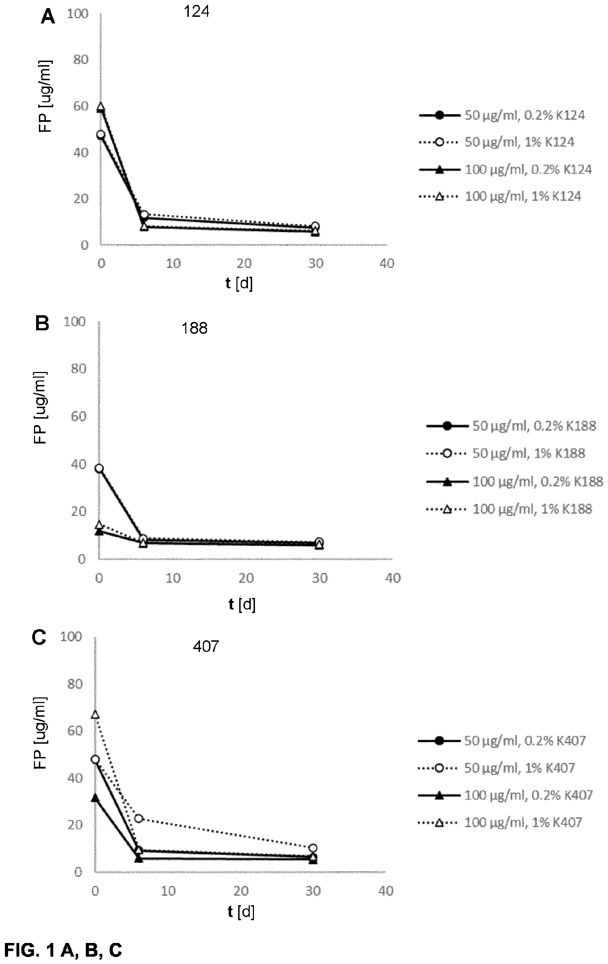

Figure 1: Determination of dissolved FP at days 0, 6 and 30 in an MS-based solvent system containing poloxamer P124 (A), P188 (B) or P407 (C), stored at 25°C;
   open/full circles = 50 *µ*g/ml FP nominal starting concentration (nsc);
   open/full triangles = 100 *µ*g/ml FP nsc; full symbols and solid lines = addition of 0.2% w/v poloxamer; open symbols and dotted lines = 1% w/v poloxamer; x-axis = storage time in days; y-axis = concentration of solubilized FP in *µ*g/ml.
Figure 2: Determination of dissolved FP in an MS-based solvent system containing poloxamer P338 (A: 2.5% w/v; B: 4%) or P407 (C: 2.5%, D: 5%) during a four-month storage period at 25°C;
   full circles = 15 *µ*g/ml FP nominal starting concentration (nsc); open circles = 25 *µ*g/ml FP nsc; full triangles = 35 *µ*g/ml FP nsc; open triangles = 50 *µ*g/ml FP nsc; x-axis = storage time in days; y-axis = concentration of solubilized FP in *µ*g/ml.
Figure 3: Determination of dissolved FP in an MS-based solvent system containing 10% P407 during a storage period of ten months at 25°C; black squares = 50 *µ*g/ml FP nsc; grey squares = 60 *µ*g/ml FP nsc; open squares = 70 *µ*g/ml FP nsc; black triangles = 80 *µ*g/ml FP nsc; grey triangles = 90 *µ*g/ml FP nsc; open triangles = 100 *µ*g/ml FP nsc; x-axis = storage time in days; y-axis = concentration of solubilized FP in *µ*g/ml
Figure 4: Determination of dose-dependent improvement of storage stability at 25°C by poloxamer P407 on 50 *µ*g/ml nsc FP in an MS-based solvent system;
   open circles grey/dotted line = 0% P407; open circles black/dashed line = 1% w/v P407; full circles, light grey = 2.5% P407; full circles, dark grey = 5% P407; full circles, black = 10% P407;
   x-axis = storage time in months; y-axis = concentration of solubilized FP in *µ*g/ml
Figure 5: Comparision of different MS-based solvent systems with regard to solubility and storage stability of 70 *µ*g/ml FP nsc at 25°C;
   full circles, black = MS-based solvent system + 10% P407;
   open circles = MS-based solvent system without saponins and without dexpanthenol, + 10% P407;
   full circles, grey = MS-based solvent system, without P407; x-axis = storage time in months; y-axis = concentration of solubilized FP in *µ*g/ml.

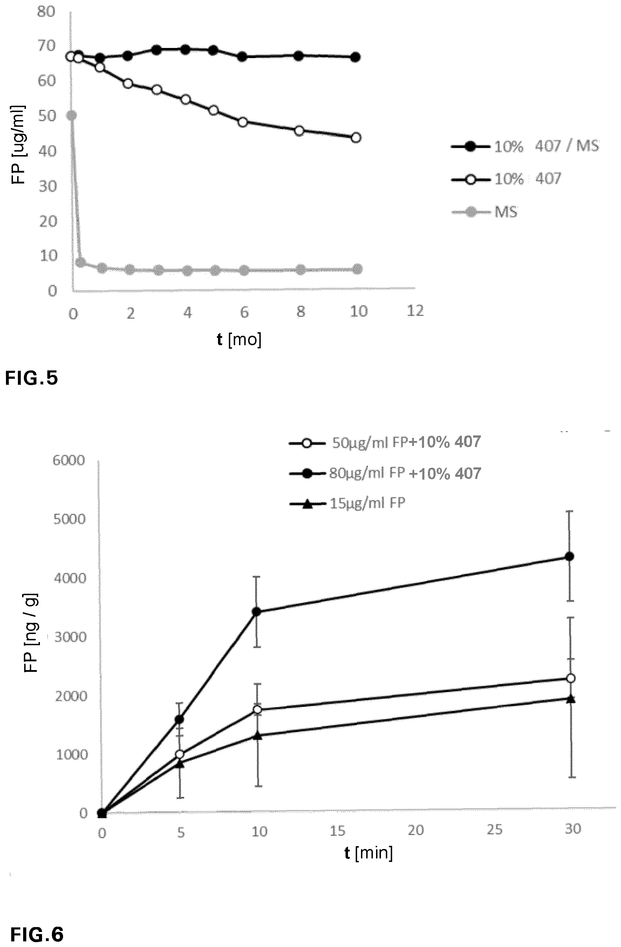

Figure 6: Evaluation of dose-dependent FP permeation into porcine nasal mucosa; determination of FP concentration in the mucosal tissue after treatment with 0.5 ml of MS solvent supplemented with 10% P407 and comprising 15.0, 50.0, or 80.0 *µ*g/mL FP dissolved therein;
   triangles = 15.0 *µ*g/mL FP (corresponding to 7.5 *µ*g solubilized FP /g tissue); open circles = 50.0 *µ*g/mL (corresponding to 25 *µ*g solubilized FP /g tissue); full circles = 80.0 *µ*g/mL FP (corresponding to 40 *µ*g solubilized FP /g tissue); y-axis = concentration of FP recovered from mucosal tissue in ng/g tissue; x-axis = permeation time in minutes; data points represent the mean of two experiments each.

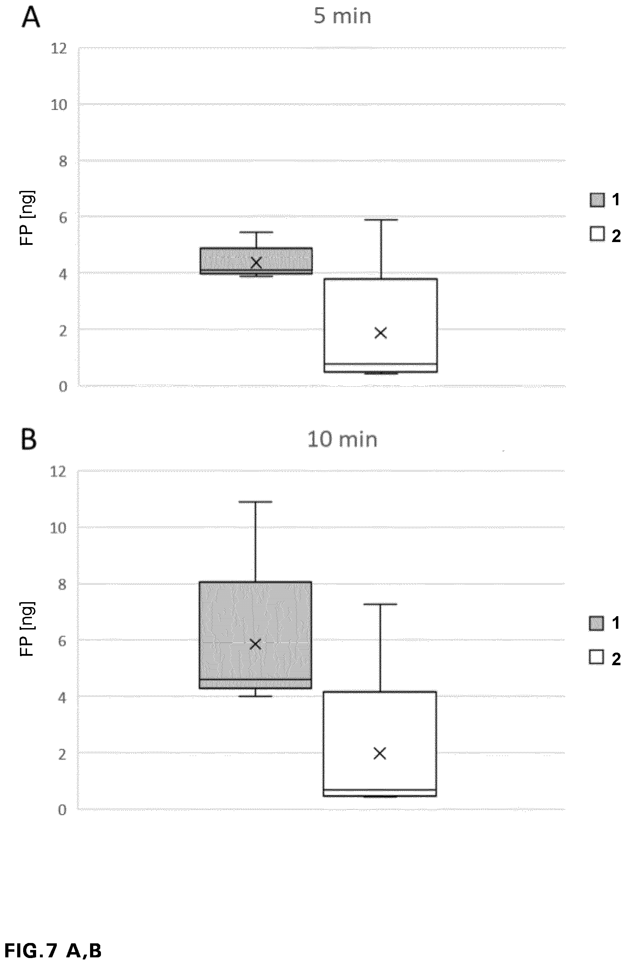

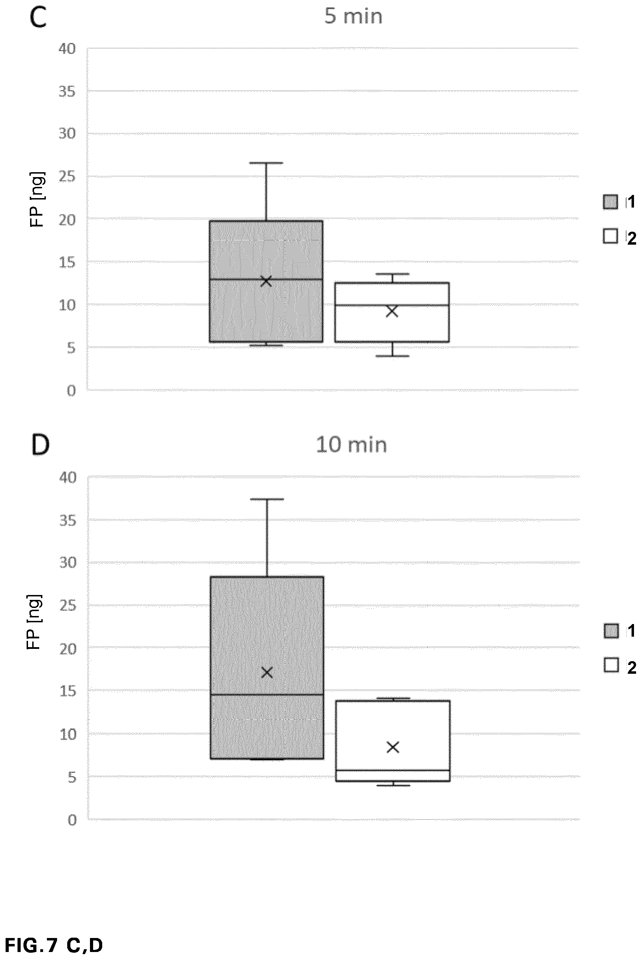

Figure 7: Permeation of FP into buccal and lingual tissue, depicted as recovered amount of FP per 6 mm punch; treatment of tissue samples with MS solvent system supplemented with 10% w/v of P407 and comprising 70 *µ*g/ml FP dissolved therein, or with 500 *µ*g/ml micronized FP suspended in Ringer solution; incubation of treated tissue samples for 5 or 10 minutes at 25°C;
   A and B: buccal samples, C and D: lingual samples; A, C incubation for 5 minutes; B, D incubation for ten minutes;
   1 grey boxes = incubation with 70 *µ*g/ml FP in MS/P407; 2 white boxes = incubation with 500 *µ*g/ml FP in Ringer solution; lines inside the boxes = medians, "x" = mean values; y-axis = FP in ng/6 mm tissue punch.

In the subsequent examples, the solubility and storage stability of fluticasone propionate (FP) was investigated with various aqueous solvent systems with and without poloxamer and compared with a solvent system prepared in accordance with the teaching of WO2017009480 (designated herein "MS solvent system"). The selected poloxamers were applied in various concentrations and their effects on the solubility and storage stability of FP evaluated.

### EXAMPLE 1

As indicated above, by using an aqueous solvent system disclosed in WO2017009480 it was possible to increase the solubility of FP from less than 0.2 *µ*g/ml in pure water up to 15 *µ*g/ml in a buffered aqueous solvent system comprised of the ingredients listed in Table 1.

**Table 1: Solvent composition MS (in accordance with WO2017009480 )**

| **Ingredient** | **Concentration** |
|---|---|
| Citric acid monohydrate | 0.5 -1 mg/mL, e.g. 0.654 mg/mL |
| Disodium hydrogen phosphate dihydrate | 1.5. - 3.5 mg/mL, e.g. 2.532 mg/mL pH adjusted to 4-8, e.g. pH 5.5 |
| Dexpanthenol | 10 - 50 mg/mL, e.g. 20 mg/mL |
| Dipotassium glycyrrhizinate | 5 - 50 mg/mL, e.g. 50 mg/mL |
| Alpha-Escin | 0.1 - 5 mg/mL, e.g. 0.3 mg/mL |
| Disodium dihydrate edetate (EDTA) | 0.2 - 2 mg/mL, e.g. 1 mg/mL |
| Propylene glycol | 1-15 %v/v, e.g. 10% (103.6 mg/mL) |
| Water for injection | Ad 1 mL |

Instead of or in addition to alpha-escin, glycyrrhizin and/or Quillaya saponaria extract may be used as saponin components. The aqueous solvent system disclosed in Table 1 shall be referred to as "MS" hereinafter.

Poloxamers have different sizes and different ratios of PEO vs PPO blocks and as such, each poloxamer has a different potential of solubilization of a certain compound. Thus, in order to find potentially suitable poloxamers a large number of candidate compounds was screened and eventually a selection of four poloxamer species was made: poloxamers nos 124, 188, 338 and 407, hereinafter designated P124, P188, P138 and P407.

In a first experimental set-up it was investigated whether and to what extent each one of the four different poloxamers if combined with the buffered aqueous MS solvent system of Table 1 would affect the solubility of FP. Low amounts of 0.2 and 1% w/v, respectively, of the poloxamers P124, P188 and P407 were added, each one individually, to the MS solvent system, while FP was added at final concentrations of 50 and 100 *µ*g/ml FP, respectively. The solubilization results are shown in Fig. 1.

No poloxamer-dose-dependent effect on FP-solubilization was observed with poloxamers P124 and P188 but the extent of solubilization of FP was different (Figure 1 A and B).
a) P124: at time point zero, i.e. immediately after mixing of the ingredients, a recovery of 95 ± 1% was analytically determined for the 50 *µ*g/ml FP formulation and only 60 ± 1% for the 100 *µ*g/ml FP formulation, irrespective of the applied concentrations of poloxamer P124.
b) P188: In contrast, a recovery of only 76.5 ± 0.5% was observed for the 50 *µ*g/ml FP formulation and only 13 ± 2% for the 100 *µ*g/ml FP formulation at both concentrations of poloxamer P188.

Neither of the two poloxamers yielded stable solubilization of FP; the concentration of dissolved FP dropped to levels below 15 *µ*g/ml within the first week of storage at room temperature, i.e. 25°C (Figures 1 A, 1B).
c) P407: interestingly, and in contrast to the results in the preceding experiments, a dose-dependent effect on the solubilization of FP was observed when P407 was added to the aqueous compositions (Fig. 1C). In the compositions containing 100 *µ*g/ml FP, the drug recovery rate on day 0 was determined as 31.8% at a poloxamer concentration of 0.2% w/v, whereas at a concentration of 1% P407 the recovery rate had more than doubled, i.e. yielding 67.4% of dissolved FP. When testing FP solubility at 50 *µ*g/ml more than 95% of the drug was dissolved already at 0.2% P407 and a further enhancement was therefore not possible by increasing the P407 concentration. However, a slightly improved 7-day storage stability was seen with the composition containing 50 *µ*g/ml FP and 1% P407 (Figure 1 C), i.e. almost 50% of the initially dissolved drug was still dissolved after 7 days.

### EXAMPLE 2

In this example, P407 and P338 were tested for their impact on the solubility and storage stability of FP in the MS solvent system of Example 1.

The poloxamers were applied at: 2.5 and 4%w/v P338; or 2.5 and 5%w/v P407; and FP was added at 15, 25, 35, or 50 *µ*g/ml.

The upper concentration of P338 was kept at or below 4% because higher concentrations caused unpleasant sensations to the nose when applied intranasally.

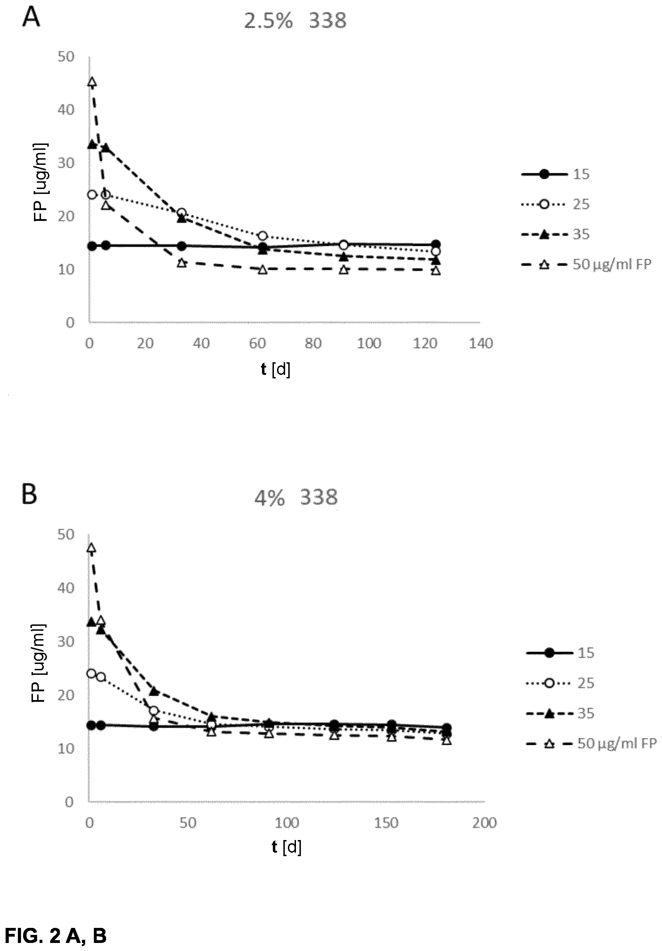

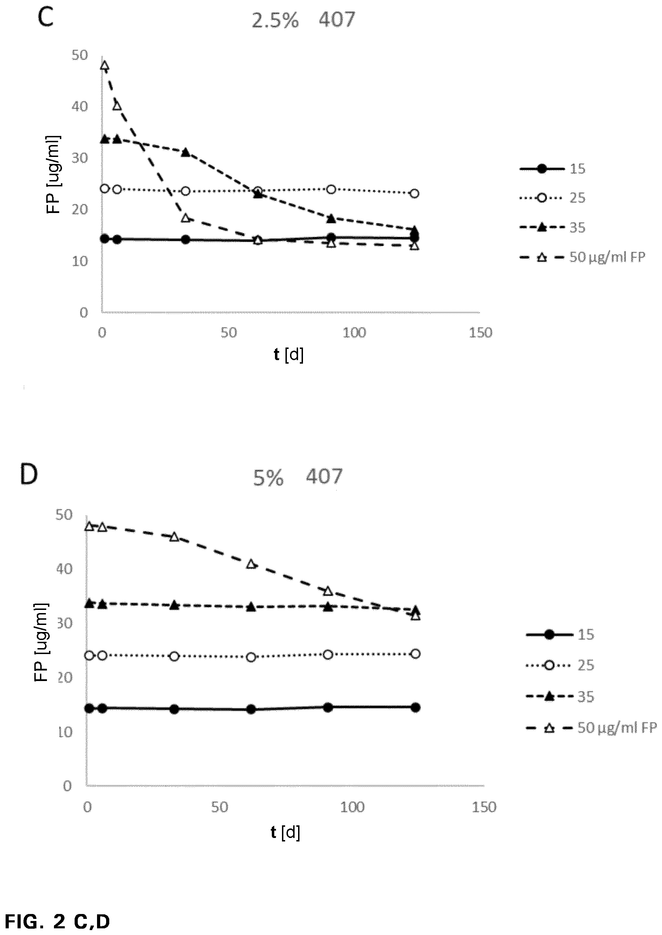

As can be taken from Figures 2A through 2D, more than 90% of the theoretically dissolved FP concentration was recovered on day 0 in the presence of 2.5 % w/v poloxamer, whether P338 or P407, and even more than 95% in the presence of 4% w/v (P338) or 5% w/v (P407) of the poloxamer component.

Also, the compositions comprising the lowest FP concentrations, i.e. 15 *µ*g/ml, remained stable over the full observation period of more than 120 days.
a) P338: However, at increased FP concentrations above 15 *µ*g/ml and using P338 as a potential solubilization enhancer, the compositions proved unstable over time and the FP concentrations dropped to the 15 *µ*g/ml level within 60 days of storage at 25°C. The higher the starting concentration of FP the faster the decrease of dissolved drug in the aqueous system, the FP concentration falling even below 15 *µ*g/ml (Fig. 2A, Fig. 2B). A dose-dependent effect was not visible with this poloxamer.
b) P407: On the other hand, a dose-dependent effect was clearly seen with P407 under otherwise identical experimental conditions. The compositions comprising starting concentrations of either 15 or 25 *µ*g/ml FP remained stable at room temperature (25°C) throughout the full observation period of more than 120 days. Whereas compositions comprising higher starting concentrations of FP remained stable only at higher poloxamer concentrations.

More specifically, in the presence of 2.5% P407 the compositions comprising 35 *µ*g/ml FP as the starting concentration remained stable over a period of about 35 days and only thereafter the recoverable FP concentration gradually dropped to the 15 *µ*g/ml level. Raising the FP starting concentration to 50 *µ*g/ml resulted in a substantially faster decrease in dissolved drug, starting already on the first day of storage.

Surprisingly, raising the P407 concentration to 5% w/v had in effect that also the compositions comprising a starting concentration of 35 *µ*g/ml FP remained stable over the full observation term of more than 120 days (Figure 2D). Whereas the compositions comprising 50 *µ*g/ml of FP remained fairly stable within the first month and only thereafter exhibited an almost linear decrease in dissolved drug over time.

### EXAMPLE 3

The experiments of Example 2 were repeated using P407 as the sole poloxamer ingredient. More specifically, it was intended to assess the entire potential of P407 on the solubilization and storage stability of FP in the aqueous MS solvent system.

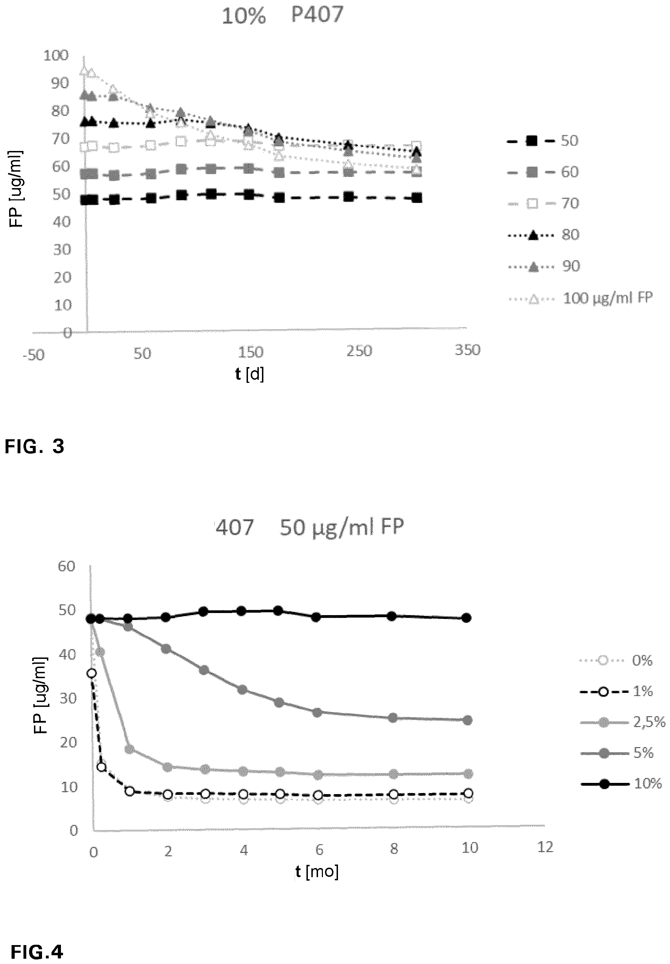

Given the promising results obtained with the addition of 5% P407 to the MS solvent system, the poloxamer concentration was raised to 10% w/v. Concomitantly, the FP starting concentration was also raised to 50 and 100 *µ*g/ml FP, respectively. As can be taken from Figure 3, 95 ± 1% of the nominal FP starting concentrations was indeed detected using HPLC analysis. The experimental compositions comprising up to 70 *µ*g/ml of dissolved FP remained stable over the course of ten months at room temperature (25°C).

The dose-dependent influence of P407 on the solubility and stability of FP in the experimental aqueous solutions was tested with formulations based on the MS solvent system and containing 50 *µ*g/ml FP (see Figure 4). The concentrations of P407 in the experimental samples were set to 0, 1, 2.5, 5 and 10% (w/v). The resulting data, pooled from two sets of experiments, clearly indicate a beneficial effect of P407 on the solubility and stability of 50 *µ*g/ml FP in the aqueous solvent system in a concentration-dependent manner.

Best results were achieved with MS-based compositions comprising 10% P407 and up to 70 *µ*g/ml of FP, resulting in unimpaired storage stability of the sample preparations over ten months at room temperature (Figures 3 and 4). P407 applied at concentrations of less than 2.5% w/v seems to have no measurable influence on the storage stability of solubilized FP and hence does not add value to an identical formulation without P407.

### EXAMPLE 4

The experiments of Example 3 were repeated with the following modifications: The nominal starting concentration of FP was adjusted to 70 *µ*g/ml and the aqueous solvent system split into three different solutions, namely:
a) the aqueous buffered MS solvent system comprising the ingredients as disclosed in Table 1, without poloxamer;
b) the aqueous buffered MS solvent system comprising the ingredients disclosed in Table 1 without the saponin and dexpanthenol components, and with 10% w/v of P407;
c) the aqueous buffered MS solvent system comprising the ingredients as disclosed in Table 1, with 10% w/v of P407.

As can be taken from Fig. 5, using solely the MS solvent system, i.e. without poloxamer, had in effect that the nominal FP starting concentration of 70 *µ*g/ml was not reached. Moreover, the preparation was rather unstable and deteriorated quickly, the dissolved FP concentration falling below a value of 10 *µ*g/ml within the first 2 weeks.

Using the MS solvent system without saponins and dexpanthenol, and adding 10% of P407 instead, resulted in a substantial improvement of FP solubility at the beginning, as evidenced by analytical recovery of more than 95% of dissolved FP relative to the nominal FP starting concentration. However, this composition was also unstable and almost linearly deteriorated from the very beginning thereby decreasing the FP concentration by about 35 - 40% down to a level of between 40 and 45 *µ*g/ml within 10 months of storage at room temperature.

However, the third tested solvent system comprising all ingredients of the MS solvent system listed in Table 1 plus 10% w/v of P407 yielded a fully dissolved and stable FP preparation which remained clear, functional and stable over an observation period of 10 months at room temperature (see Fig. 5). It can therefore be derived from the experimental data that an aqueous buffer system adjusted to a pH of between 4 and 8, preferably pH 5.5, and comprising a combination of poloxamer P407 and a saponin component selected from the group of alpha-escin, glycyrrhizin, and Quillaja saponaria extract, and optionally supplemented with dexpanthenol and/or an organic co-solvent such as propylene glycol, generates synergistic effects with respect to remarkably improving both the solubility and storage stability of fluticasone propionate (FP) in an aqueous solvent, as compared to aqueous FP preparations, i.e. suspensions, known in the art.

### EXAMPLE 5

Additional experimentation was performed to test the bioavailability of FP from preparations containing P407 upon delivery to the nasal mucosa. Specifically, it was investigated whether:
a) FP permeates the nasal mucosa from sprayable preparations based on the buffered MSV aqueous solvent system containing poloxamer P407; and if so, whether
b) increased concentrations of dissolved FP, e.g. of 50 or 80 *µ*g/ml, in the sprayable preparations inevitably yield an improvement in FP permeation into the mucosal tissue, as compared to lower FP concentrations of e.g.15 *µ*g/ml FP as achievable with the MS solvent system of Table 1 in the absence of poloxamer.

In an ex-vivo experiment punches of freshly isolated porcine nasal mucosa were incubated with different FP-containing preparations, thereafter washed, weighed and snap-frozen. The following preparations were analyzed:
- 15 *µ*g/ml FP in the MS solvent system
- 50 *µ*g/ml FP in the MS solvent system supplemented with 10% w/v P407
- 80 *µ*g/ml FP in the MS solvent system supplemented with 10% w/v P407

The FP-content in the experimental porcine nasal mucosa samples was determined by an external analytical institute (Pharmacelsus GmbH, Saarbrücken, DE) by means of high-performance liquid chromatography-mass spectrometry (HPLC-MS) analysis of homogenized sample material.

As can be seen in Figure 6, the preparations comprising the increased concentrations of FP in the presence of P407 clearly outperform the preparations based solely on the MS solvent system, i.e. without the poloxamer component. FP apparently permeates in a dose-dependent manner into porcine nasal mucosa.

### EXAMPLE 6

In a subsequent experiment it was additionally investigated whether:
a) FP is able to permeate buccal and lingual tissues from preparations referred to in Example 5, i.e., containing a mixture of the MSV solvent system and 10% w/v of poloxamer P407; and if so, whether
b) an experimental aqueous preparation comprising 70 *µ*g/ml of dissolved FP will deliver less or more FP to the buccal and lingual tissues than a commercially available aqueous preparation comprising 500 *µ*g/ml micronized FP suspended in a Ringer solution.

The ex-vivo experiments were performed on freshly isolated buccal and lingual porcine tissues by incubating the tissues with 200 *µ*l of the test sample solutions for 5 or 10 minutes at room temperature inside a muffle put tightly on the respective tissues, followed by thoroughly washing the incubated tissues with Ringer solution. Thereafter, 6 mm punches were taken from the treated tissue areas, weighed, snap-frozen and sent for FP-content analysis to Pharmacelsus GmbH. The comparative sample comprising 500 *µ*g/ml micronized FP suspended in Ringer solution was obtained from a blister of a Flixotide Diskus forte 500 microgram inhaler (Glaxo Wellcome Production, Evreux, FR)

The results depicted in Figure 7 clearly indicate that a preparation prepared in line with the present invention and comprising 70 *µ*g/ml of dissolved FP delivers substantially more drug into buccal as well as lingual tissues than a commercially available aqueous suspension comprising 500 *µ*g/ml FP. The observed effect was more pronounced with buccal tissue. Moreover, it can further be derived from this example that the permeation of FP from a solution prepared in accordance with the invention occurs in a time-dependent manner, i.e. the amount of FP found in the punches after ten minutes is higher than after five minutes, while with the comparative sample of a 500 *µ*g/ml FP suspension a plateau seems to be reached already after five minutes of incubation in both the buccal and lingual tissues.

## Claims

1. A buffered aqueous composition comprising fluticasone propionate dissolved therein, **characterized in that** it comprises
- at least one saponin component selected from the group consisting of escin, glycyrrhizin, and Quillaja saponaria extract;
- a buffer adjusted to a pH of from 4 to 8;
- poloxamer P407 in a concentration of from 2.5 to 15 %w/v; and
- fluticasone propionate in a concentration of at least 20 *µ*g/mL.

2. The buffered aqueous composition of claim 1, **characterized in that** it comprises dexpanthenol in a concentration of from 5 to 50 mg/ml.

3. The buffered aqueous composition of claim 1 or 2, **characterized in that** it comprises propylene glycol in a concentration of from 20 to 150 mg/ml.

4. The buffered aqueous composition of any one of claims 1 to 3, **characterized in that** it comprises EDTA in a concentration of from 0.5 to 3 mg/ml.

5. The buffered aqueous composition of any one of claims 1 to 4, **characterized in that** it comprises a citrate buffer or a phosphate buffer or a mixture of both buffers.

6. The buffered aqueous composition of any one of claims 1 to 5, **characterized in that** it comprises at least from 30 to 90 *µ*g/mL of fluticasone propionate dissolved therein.

7. The buffered aqueous composition of any one of claims 1 to 6, for use as a medicament.

8. The buffered aqueous composition of claim 7, for use in the treatment of inflammatory and/or allergic conditions of a human individual.

9. The buffered aqueous composition of claim 8, for use in the treatment of inflammatory and/or allergic conditions affecting the respiratory pathways, the lungs, the mouth, the nose, the eyes, mucosal tissues of the genital including the anogenital region, and/or the skin.

10. The buffered aqueous composition of claim 9, for use in the treatment of acute, chronic, or allergic asthma, hayfever, allergic diseases caused by pollen, animal hair or dust particles, as well as eye inflammations including conjunctivitis, and skin inflammations including skin inflammations caused by lichen ruber planus or lichen sclerosus.

11. The buffered aqueous composition of claim 8 adapted for topical application in a form selected from the goup consisting of a liquid solution including a lotion, a nasal spray, a mouth spray, a gargling solution, and eye drops, a gel, and an ointment.
